# EUROPEAN PATENT APPLICATION

(11) **EP 4 431 107 A1**
(43) Date of publication of application: **18.09.2024**
(21) Application number: 22900580.6
(22) Date of filing: 01.12.2022
(51) Int. Cl.: A61K 36/752, A61K 36/71, A61K 36/538, A61K 36/484, A61P 25/02, A61P 25/04

(54) **APPLICATION OF TRADITIONAL CHINESE MEDICINE COMPOSITION IN PREPARATION OF DRUGS FOR TREATING NEURITIS**

(30) Priority: 03.12.2021 CN 202111468002
(71) Applicant: Shandong New Time Pharmaceutical Co., Ltd., Linyi City, Shandong 273400 (CN)
(72) Inventor: ZHANG, Guimin, Linyi, Shandong 276006 (CN); SUN, Chenglei, Linyi, Shandong 276006 (CN); YAO, Jingchun, Linyi, Shandong 276006 (CN); GUAN, Yongxia, Linyi, Shandong 276006 (CN); MA, Qingwen, Linyi, Shandong 276006 (CN)
(74) Representative: Handley, Matthew Edward
(86) International application number: PCT/CN2022/135766
(87) International publication number: WO 2023/098776

(57) **Abstract**

A traditional Chinese medicine composition for treating neuritis. The traditional Chinese medicine composition is prepared mainly from Notopterygii Rhizoma et Radix, Angelicae Pubescentis Radix, Poria, Saposhnikoviae Radix, Schizonepetae Herba, Chuanxiong Rhizoma, Platycodonis Radix, Bupleuri Radix, Peucedani Radix, Aurantii Fructus, and Glycyrrhizae Radix et Rhizoma. The traditional Chinese medicine composition can significantly relieve facial paralysis, movement disorders, and other related symptoms of facial neuritis in a rat, remarkably improve expression of VEGFb, VEGFR1, and VEGFR2 proteins in postoperative side facial nerves of the rat, and promote recovery and regeneration of damaged rear axons.

## Description

### Technical Field

The present invention pertains to the technical field of traditional Chinese medicine, and relates to a traditional Chinese medicine composition and its use, in particular to a traditional Chinese medicine composition and its use in preparing drugs for treating neuritis.

### Background

Neuritis refers to peripheral nerve disease, which is an organic disease of peripheral nervous system. According to the location and function of nerves, the nervous system can be divided into central nervous system and peripheral nervous system. Among them, facial neuritis is also called facial paralysis or facioplegia, which is facial muscle paralysis, mainly manifested as facial muscle movement obstruction. The clinical manifestations of facial paralysis are mainly bilateral facial paralysis with one severe side and one mild side and unilateral facial paralysis.

In traditional Chinese medicine, it is called facial distortion. It is due to the lack of healthy qi and poor collaterals of human body, and wind-evil takes advantage of weakness to enter Yangming vein of middle head and face, which leads to disharmony between nutrient qi and defensive qi on one side of face, obstruction of qi and blood, and loss of nourishment of meridians. It is divided into wind-cold syndrome and wind-heat syndrome.

The symptoms of wind-cold syndrome are sudden onset, skewed mouth, incomplete eyelid closure, fear of wind and aversion to cold, headache, stuffy nose, tight facial muscles, sore muscles and joints, reddish tongue, thin white fur and floating and tense pulse. The treatment method is to dispel wind and cold, eliminate phlegm and dredge collaterals. The main method is to apply Sanbai Wuchong Decoction: Paeonia lactiflora pallas 20g, Angelica dahurica 15g, Rhizoma typhonii 6g, Bombyx batryticatus 15g, Cicada slough 15g, Fried earthworm 15g, Scorpio 10g, Centipede 2 (packed separately), Saposhnikoviae Radix 10g, Chuanxiong Rhizoma 10g, Uncaria rhynchophylla 20g, Astragalus membranaceus 30g. Decoct the above medicine in water twice except centipede, take it separately in the morning and evening, and take it once a day. Bake the centipede on tile, grind it into fine powder, and take it with water twice. In the prescription, Saposhnikoviae Radix, Angelica dahurica, Rhizoma typhonii and Uncaria rhynchophylla can dispel wind and cold, and dredge collaterals, promote blood circulation and nourish nutrient qi, and "five insects" (i.e., centipede, scorpion, Bombyx batryticatus, Cicada slough and earthworm) can expel wind and dredge collaterals; Paeonia lactiflora pallas and Chuanxiong Rhizoma can nourish blood, promote blood circulation and dispel wind; Astragalus membranaceus can tonify qi, strengthen body resistance and dispel wind, and all medicines are combined to eliminate pathogen and strengthen vital qi, eliminate wind and cold, nourish meridians, smooth blood vessels and heal facial paralysis.

The symptoms of wind-heat syndrome are sudden onset, skewed mouth and eyes, headache, hot face or fever, aversion to wind, upset, bitter taste, pain behind ears, dry mouth and sore throat, dry stool, yellow urine, red tongue tip, thin yellow fur and floating and tense pulse. The treatment method is to dispel wind and clear heat, promote blood circulation and dredge collaterals. The main method is to apply Jiawei Qianzheng Powder: Scorpion 7g, Rhizoma typhonii 7g, Bombyx batryticatus 7g, Centipede 2, Earthworm 7g, Angelica sinensis 10g, Paeonia lactiflora pallas 10g, Chuanxiong Rhizoma 10g, Saposhnikoviae Radix 10g, Uncaria rhynchophylla 10g, Forsythia suspensa 15g, Radix isatidis 15g and Folium Isatidis 15g. In the prescription, with pungent flavor, warmness and summer-heat attributing to rise and dispersion, Rhizoma typhonii is a monarch drug for dispelling wind, eliminating phlegm and dredging collaterals and treating the wind of head and face; Scorpion, Bombyx batryticatus and earthworm can dispel wind and stop spasm, among which Bombyx batryticatus has the function of eliminating phlegm, and Scorpion is good at dredging collaterals; Centipede and Uncaria rhynchophylla can dispel wind and dredge collaterals; Angelica sinensis, Paeonia lactiflora pallas, Chuanxiong Rhizoma and Saposhnikoviae Radix can dispel wind and dredge collaterals, promote blood circulation and nourish nutrient qi, taking the meaning of "to cure wind, blood circulation shall be promoted first, and wind will be cured after good blood circulation", and Forsythia suspensa, Radix isatidis and Folium Isatidis can clear heat, eliminate dampness and detoxify. The above medicines are combined to go through the meridians of the head and face, and reach the sick place directly to eliminate pathogen and strengthen vital qi.

CN108201592A discloses an oral traditional Chinese medicine preparation for treating facial paralysis caused by neuritis. The raw materials and parts by weight of the traditional Chinese medicine are Bupleuri Radix 30g, Scutellaria baicalensis 17g, Schizonepetae Herba 14g, Saposhnikoviae Radix 15g, Rhizoma typhonii 15g, Gastrodia elata 15g, Scorpio 15g, Glycyrrhizae Radix et Rhizoma 10g, Garden balsam stem 15g, Notopterygii Rhizoma et Radix 20g and Earthworm 20g. Although Gastrodia elata has a good effect of dredging meridians and activating collaterals, it has great toxic and side effects. In addition, Garden balsam stem has strong irritation, which is easy to stimulate intestines and stomach and damage gastric mucosa. The traditional Chinese medicine preparation provided by the present patent has a long treatment period, and the disease can be basically cured only after 11 courses of treatment.

At present, there are reports of treating facial neuritis with the modified prescription of Schizonepetae Herba and Saposhnikoviae Radix toxin-vanquishing powder.

The 5th edition of China News of Traditional Chinese Medicine published the modified prescription of Schizonepetae Herba and Saposhnikoviae Radix toxin-vanquishing powder to treat facial paralysis of meridian type stroke in wind-cold attack. First diagnosis: Schizonepetae Herba 10g, Saposhnikoviae Radix 10g, Bupleuri Radix 12g, Scutellaria baicalensis 10g, Notopterygii Rhizoma et Radix 10g, Angelicae Pubescentis Radix 10g, Chuanxiong Rhizoma 12g, Mint 10g, Honeysuckle 10g, Forsythia suspensa 10g, Angelica dahurica 10g, Pueraria lobata 20g, Hedyotis diffusa 15g, Red paeony root 10g, Great burdock achene 10g and Glycyrrhizae Radix et Rhizoma 9g. 2 doses. One dose a day in three times. Second diagnosis: Angelicae Pubescentis Radix and Angelica dahurica were removed from the original prescription, and Angelica sinensis 10g, Cicada slough 6g, Rhizoma cimicifugae 10g, Great burdock achene 10g and Acorus gramineus 10g were added. 2 doses. Third diagnoses: Red paeony root 10g, Chuanxiong Rhizoma 6g, Angelica sinensis 10g, Earthworm 10g, Astragalus membranaceus 20g, Pueraria lobata 20g, Rhizoma cimicifugae 10g, Cicada slough 9g, whole worm 6g, Radix clematidis 20g, Hedyotis diffusa 15g, Bombyx batryticatus 10g, Centipede 1 and Glycyrrhizae Radix et Rhizoma 6g. 2 doses. One dose a day in three times. Fourth diagnoses: The above prescription is slightly adjusted, and 2 doses are used for prognosis. After 2 doses, there was no air leakage in the left corner of the mouth and no discomfort in the face. All the diseases were cured, and 8 doses were taken in total. In this report, the modified prescription of Schizonepetae Herba and Saposhnikoviae Radix toxin-vanquishing powder was selected and the disease was treated by stages according to syndrome differentiation. Although the treatment period was short, the prescription was changed every 2 doses, which increased the number of patients' inquiries and increased the probability of wind-cold attack.

JINGFANG Preparation is a modern ready-for-use traditional Chinese medicine preparation developed according to Schizonepetae Herba and Saposhnikoviae Radix toxin-vanquishing powder, which is prepared from Notopterygii Rhizoma et Radix, Angelicae Pubescentis Radix, Poria, Saposhnikoviae Radix, Schizonepetae Herba, Chuanxiong Rhizoma, Platycodonis Radix, Bupleuri Radix, Peucedani Radix, Aurantii Fructus and Glycyrrhizae Radix et Rhizoma. Saposhnikoviae Radix, Schizonepetae Herba and Notopterygii Rhizoma et Radix is pungent-warm diaphoresis, which can dispel wind-cold, Bupleuri Radix can strengthen relief of exterior syndrome, Angelicae Pubescentis Radix can dispel wind and eliminate dampness, Chuanxiong Rhizoma can promote blood circulation and dispel wind, and Aurantii Fructus can regulate qi by alleviation of mental depression. The synergy of these drugs has the functions of dispelling wind and relieving exterior syndrome, promoting blood circulation and dredging collaterals, eliminating phlegm and killing toxin, clearing heat and eliminating dampness. At present, there is no report on the treatment of facial neuritis with JINGFANG Preparation.

### Summary

The present invention is a further development of the use of the existing products "JINGFANG KELI" and "JINGFANG HEJI". The new application comes from the feedback from employees of the company. Employees respond: After facial paralysis symptoms occur due to long-term air conditioning, they take JINGFANG KELI daily to prevent colds during routine treatment of facial neuritis, and find that facial paralysis symptoms are obviously relieved and the treatment period of facial neuritis is obviously shortened. Therefore, according to the feedback from employees, the applicant made further research on the use of JINGFANG Preparation in treating neuritis, especially facial neuritis.

The first purpose of the present invention is to provide a traditional Chinese medicine composition for treating neuritis, which comprises Notopterygii Rhizoma et Radix, Angelicae Pubescentis Radix, Poria, Saposhnikoviae Radix, Schizonepetae Herba, Chuanxiong Rhizoma, Platycodonis Radix, Bupleuri Radix, Peucedani Radix, Aurantii Fructus and Glycyrrhizae Radix et Rhizoma.

Further, the traditional Chinese medicine composition comprises the following raw materials:
Schizonepetae Herba 50-100 parts by weight, Saposhnikoviae Radix 50-100 parts by weight, Notopterygii Rhizoma et Radix 50-100 parts by weight
Angelicae Pubescentis Radix 50-100 parts by weight, Bupleuri Radix 50-100 parts by weight, Peucedani Radix 50-100 parts by weight
Chuanxiong Rhizoma 50-100 parts by weight, Aurantii Fructus 50-100 parts by weight, Poria 50-100 parts by weight
Platycodonis Radix 50-100 parts by weight, and Glycyrrhizae Radix et Rhizoma 5-50 parts by weight.

Preferably, the traditional Chinese medicine composition comprises the following raw materials:
Schizonepetae Herba 75 parts by weight, Saposhnikoviae Radix 75 parts by weight, Notopterygii Rhizoma et Radix 75 parts by weight
Angelicae Pubescentis Radix 75 parts by weight, Bupleuri Radix 75 parts by weight, Peucedani Radix 75 parts by weight
Chuanxiong Rhizoma 75 parts by weight, Aurantii Fructus 75 parts by weight, Poria 75 parts by weight
Platycodonis Radix 75 parts by weight, and Glycyrrhizae Radix et Rhizoma 25 parts by weight.

The neuritis includes facial neuritis, trigeminal neuritis and polyneuritis.

The neuritis is caused by one or more of infection, poisoning, genetic defect, nutritional disorder, immune damage, metabolic disorder, endocrine disorder, congenital malformation, blood circulation disorder, abnormal hyperplasia, wind-cold and trauma.

Further, the traditional Chinese medicine composition can improve the symptoms of neuritis, and the symptoms include irritation symptoms and/or destruction symptoms, wherein the irritation symptoms show pain and numbness, and the destruction symptoms show weakness and paralysis.

Specifically, the neuritis is preferably facial neuritis, and the traditional Chinese medicine composition can improve the symptoms of facial neuritis, including shallow nasolabial groove, distortion of commissure, air leakage and salivation in speech, loss of facial random movements, large palpebral fissure, inability to successfully complete frowning, closing eyes, whistling and other movements, and loss of one or more facial expressions.

Further, the traditional Chinese medicine composition can be prepared with pharmaceutically acceptable excipients to form a preparation.

Specifically, the preparation comprises granules, mixtures, tablets, capsules, pills, dustpowders, powders, syrups, microcapsules and ointments, preferably JINGFANG KELI and JINGFANG HEJI.

The second purpose of the present invention is to provide the use of the JINGFANG Preparation in the preparation of a medicament for treating neuritis.

The third purpose of the present invention is to provide the use of the JINGFANG Preparation in the preparation of a medicament for treating facial neuritis.

The JINGFANG Preparation of the present invention includes but is not limited to JINGFANG KELI and JINGFANG HEJI, and the traditional Chinese medicine preparations prepared by adopting the traditional Chinese medicine composition of the present invention are all included in the JINGFANG Preparation category.

Compared with the prior art, the present invention has achieved remarkable technical effects:
(1) The traditional Chinese medicine composition of the present invention can obviously relieve facial paralysis, dyskinesia and other related symptoms of facial neuritis in rats.
(2) The traditional Chinese medicine composition of the present invention can significantly improve the expression of VEGFb, VEGFR1 and VEGFR2 proteins in lateral nerves of rats after operation, and promote the recovery and regeneration of axons after injury.
(3) The traditional Chinese medicine composition of the present invention can stimulate the electrical conduction of the facial nerve of rats and improve the electrical conduction speed of the facial nerve.
(4) The traditional Chinese medicine composition of the present invention can reduce the expression of Beclin1 protein in the facial nerve tissue of rats, increase the level of P62 protein, participate in the regulation mechanism of autophagy, and promote the functional recovery of facial nerve.

### Brief Description of the Drawings

Fig. 1-2: Study on Nerve Conduction Velocity of Lateral Nerve Potential of Rats in Each Group after Administration for 14 Days
Fig. 3-4: Expression of Beclin1 Protein in Facial Nerve Tissue of Rats in Each Group after Administration for 14 Days
Fig. 5-6: Expression of P62 Protein in Facial Nerve Tissue of Rats in Each Group after Administration for 14 Days

### Detailed Description of the Preferred Embodiments

The summary of the present invention is described in further detail by way of examples in order to make the purse and the technical proposal of the present invention clearer, but it should not be understood that the scope of the above subject area of the present invention is limited to the following examples. Corresponding substitutions or modifications made in accordance with ordinary knowledge and conventional means in the art without departing from the above-mentioned techniques of the present invention are included in the present invention.

### Example 1 Preparation of granule (commercially available JINGFANG KELI)

### Prescription:

Schizonepetae Herba 75g, Saposhnikoviae Radix 75g, Notopterygii Rhizoma et Radix 75g, Angelicae Pubescentis Radix 75g, Peucedani Radix 75g
Chuanxiong Rhizoma 75g, Aurantii Fructus 75g, Poria 75g, Platycodonis Radix 75g, and Glycyrrhizae Radix et Rhizoma 25g;

### Preparation method:

Among the above eleven herbs, extract volatile oils of Schizonepetae Herba, Saposhnikoviae Radix, Notopterygii Rhizoma et Radix, Angelicae Pubescentis Radix, Peucedani Radix, Chuanxiong Rhizoma and Aurantii Fructus respectively, and collect the distilled aqueous solution of Chuanxiong Rhizoma and Aurantii Fructus separately; percolate the residues of Chuanxiong Rhizoma, Aurantii Fructus and Poria with 25% ethanol solution prepared from the above aqueous solution as solvent according to the percolation method under the terms of fluid extract and extract; decoct the residues of Schizonepetae Herba, Saposhnikoviae Radix, Notopterygii Rhizoma et Radix, Angelicae Pubescentis Radix and Peucedani Radix together with the rest of Bupleuri Radix, Platycodonis Radix and Glycyrrhizae Radix et Rhizoma in water for 1.5 hours twice, combine decoction, filter, and concentrate filtrate into thick paste; combine the percolated liquid and the thick paste, mix evenly, stand, filter, concentrate the filtrate into clear paste with a relative density of 1.30 (80-85°C), take 1 part of the clear paste, add 6 parts of sucrose, mix evenly to make granules, dry, add the volatile oils such as Schizonepetae Herba, and mix evenly.

### Example 2 Preparation of granule

### Prescription:

Schizonepetae Herba 50g, Saposhnikoviae Radix 50g, Notopterygii Rhizoma et Radix 50g, Angelicae Pubescentis Radix 50g, Peucedani Radix 50g
Chuanxiong Rhizoma 50g, Aurantii Fructus 50g, Poria 50g, Platycodonis Radix 50g, and Glycyrrhizae Radix et Rhizoma 5g.
Preparation method: Same as Example 1.

### Example 3 Preparation of granule

### Prescription:

Schizonepetae Herba 100g, Saposhnikoviae Radix 100g, Notopterygii Rhizoma et Radix 100g, Angelicae Pubescentis Radix 100g, Peucedani Radix 100g
Chuanxiong Rhizoma 100g, Aurantii Fructus 100g, Poria 100g, Platycodonis Radix 100g, and Glycyrrhizae Radix et Rhizoma 50g.
Preparation method: Same as Example 1.

### Example 4 Preparation of mixture (commercially available JINGFANG HEJI)

### Prescription:

Schizonepetae Herba 97g, Saposhnikoviae Radix 97g, Notopterygii Rhizoma et Radix 97g, Angelicae Pubescentis Radix 97g, Peucedani Radix 97g
Chuanxiong Rhizoma 97g, Aurantii Fructus 97g, Poria 97g, Platycodonis Radix 97g, and Glycyrrhizae Radix et Rhizoma 32.4g;

### Preparation method:

Distill Schizonepetae Herba, Saposhnikoviae Radix, Notopterygii Rhizoma et Radix, Angelicae Pubescentis Radix, Aurantii Fructus, Chuanxiong Rhizoma and Peucedani Radix and extract volatile oils respectively, and collect the distilled aqueous solution separately; percolate the residues of Chuanxiong Rhizoma, Aurantii Fructus and Poria with 25% ethanol solution prepared from the above aqueous solution as solvent according to the percolation method under the terms of fluid extract and extract, collect percolate and recover ethanol under reduced pressure. Decoct the remaining five kinds of residues with Bupleuri Radix, Platycodonis Radix and Glycyrrhizae Radix et Rhizoma in water for three times, filter, combine filtrate, concentrate to about 1300mL, combine with percolate, stand still, filter, concentrate to about 1000mL, add 3g of sodium benzoate and the above volatile oil, stir evenly, and add water to make it 1000mL.

### Comparative example 1: Decoction

Bupleuri Radix 30g, Scutellaria baicalensis 17g, Schizonepetae Herba 14g, Saposhnikoviae Radix 15g, Rhizoma typhonii 15g, Gastrodia elata 15g
Scorpion 15g, Glycyrrhizae Radix et Rhizoma 10g, Garden balsam stem 15g, Notopterygii Rhizoma et Radix 20g, Earthworm 20g

### Preparation method:

Prepare decoction to 500mL by conventional decoction.

### Comparative example 2: Decoction

Schizonepetae Herba 10g, Saposhnikoviae Radix 10g, Bupleuri Radix 12g, Scutellaria baicalensis 10g, Notopterygii Rhizoma et Radix 10g, Angelicae Pubescentis Radix 10g, Chuanxiong Rhizoma 12g
Mint 10g, Honeysuckle 10g, Forsythia suspensa 10g, Angelica dahurica 10g, Pueraria lobata 20g, Hedyotis diffusa 15g
Red paeony root 10g, Great burdock achene 10g and Glycyrrhizae Radix et Rhizoma 9g
Preparation method: Prepare decoction to 500mL by conventional decoction.

### Comparative example 3: Decoction

Schizonepetae Herba 10g, Saposhnikoviae Radix 10g, Bupleuri Radix 12g, Scutellaria baicalensis 10g, Notopterygii Rhizoma et Radix 10g, Chuanxiong Rhizoma 12g, Angelica sinensis 10g
Mint 10g, Honeysuckle 10g, Forsythia suspensa 10g, Pueraria lobata 20g, Hedyotis diffusa 15g, Cicada slough 6g
Red paeony root 10g, Great burdock achene 10g, Glycyrrhizae Radix et Rhizoma 9g, Rhizoma cimicifugae 10g, Great burdock achene 10g, and Acorus gramineus 10g
Preparation method: Prepare decoction to 500mL by conventional decoction.

### Pharmacological experiment

The inventor carried out related pharmacodynamic experimental research to prove the efficacy of JINGFANG Preparation in the present invention in treating facial neuritis. It should be noted that the following experimental studies are carried out on the basis of proving drug safety by acute toxicity test and long-term toxicity test, and the dosage in the experimental studies is within the safe dose range. The medicines selected in the following pharmacodynamic tests are prepared from the representative formula of the present invention; pharmacodynamic experiments have also been carried out by the inventor of the medicines prepared from other formulations contained in the present invention, and the experimental results have the same or similar effects, but due to space limitations, they are not listed here.

In addition, the following pharmacodynamic experiments only take some animal models as examples to verify the efficacy of the present invention. Here, only the pharmacodynamic experimental results of the traditional Chinese medicine composition for treating facial neuritis are displayed. The inventor has also done relevant pharmacodynamic experiments for neuritis caused by other types and other pathogenic factors mentioned in the present invention, and the experimental results show the same or similar effects, so the pharmacodynamic experimental results will not be listed one by one.
1 Materials
1.1 Experimental drugs and reagents
1.1.1 Drugs
   Granules obtained in Examples 1, 2 and 3 of the present invention;
   Example 4 Mixture;
   Decoctions obtained in Comparative examples 1-3.
1.1.2 Dose
   Example 1 Granule: 2.02g/kg (low dose), 4.05g/kg (medium dose), 8.10g/kg (high dose);
   Example 2 Granule: 4.05g/kg;
   Example 3 Granule: 4.05g/kg;
   Example 4 Mixture: 4.05mL/kg;
   Decoction in Comparative example 1: 18mL/kg;
   Decoction in Comparative example 2: 18mL/kg;
   Decoction in Comparative example 3: 18mL/kg;
1.2 Experimental animals
   SD rats, SPF grade, 180-220g, experimental animal license number: SYXK (Lu) 20180008, provided by Lunan Pharmaceutical Group Co., Ltd., were fed adaptively under standard conditions for one week before the experiment.

2. Methods
2.1 Molding method and grouping
   A number of rats, half male and half female, were taken. An appropriate amount of ether was poured into the small beaker to completely infiltrate the cotton ball, and the small beaker was put into the rat anesthesia chamber after the ether container was tightly covered. Then, the rat is quickly put into the anesthesia chamber before the anesthesia chamber is closed tightly. When the rats fell spontaneously and fast breathing slowed down, they were taken out of the anesthesia chamber to check their corneal reflexes and pain reflexes. If the rat did not close their eyes and dodge when touching their cornea with the tip of the hemostat, it was proved that the corneal reflexes disappeared; if the rat did not struggle or dodge when clamping the toe of the rat with appropriate force with a hemostat, it was proved that the pain reflexes disappeared and the rats' general anesthesia was effective. Then, the right side of the rat was fixed upwards on the surgical table in a lateral position because the right side of the face is the surgical area to be sterilized with 1% iodophor solution. A cortex of about 1 cm long was cut at the midpoint of the line connecting the right tragus and the pupil of the right eye of the rat, with the incision perpendicular to the connecting line. After the subcutaneous tissue of the incision was separated to the muscular surface, the facial nerve was separated along the exposed buccal branch of the rat facial nerve towards the right auricle of the rat until the right facial nerve stem of the rat was exposed. The facial nerve was separated and clamped with the front end of the needle holder for 90s. After the clamping damage was formed, the wound was sutured with 3-0 silk suture, and applied with erythromycin ointment to prevent infection. The modeled rats were taken to form the model group, three dose groups of Example 1 (high, medium, and low), the groups of Examples 2-4, and the groups of Comparative examples 1-3, with 10 rats in each group, half male and half female. The operation of 10 rats in the blank group (half male and half female) was basically the same as above, but the right facial nerve stem was only exposed without any injury treatment.
2.2 Administration
   Rats in each administration group are given corresponding drugs by intragastric administration, while rats in the blank group and model group are given the same amount of normal saline by intragastric administration once a day for 14 days.

3. Statistical processing
SPSS 22.0 software is used for statistical analysis of the data. The measurement data are expressed by (x̅ ± s). One-way ANOVA is used for comparison among multiple groups, and independent sample T test is used for analysis between two groups. The difference is statistically significant with P<0.05.
4 Observation indexes and experimental results
4.1 Rat behavioral score
4.1.1 Scoring criteria

**Table 1 Scoring Table of Tentacle Movement**

| Score | Tentacle movement | Position of tentacle |
|---|---|---|
| 1 | No tentacle movement | Backward |
| 2 | Mild tremor | Backward |
| 3 | Large tremor | Backward |
| 4 | Normal tentacle movement | Backward |
| 5 | Normal | Forward |

**Table 2 Observation Table of Eye Closure and Blink Reflex**

| Score | Blink reflex and eye closure |
|---|---|
| 1 | No blink reflex or eye closure |
| 2 | Ocular muscle tension with no blink reflex |
| 3 | Blink reflex with 50% eye closure |
| 4 | Blink reflex with 75% eye closure |
| 5 | Complete closure, positive blink reflex |

4.1.2 Statistical results

**Table 3 Behavioral Scores of Rats after Administration on 0d (x̅ ± s, n=10)**

| Group | | Behavioral score |
|---|---|---|
| | | 0 d |
| Blank group | | 9.79±0.51 |
| Model group | | 1.71±0.54* |
| Example 1 | High dose group | 1.68±0.44* |
| | Medium dose group | 1.73±0.49* |
| | Low dose group | 1.72±0.36* |
| Example 2 group | | 1.75±0.46* |
| Example 3 group | | 1.69±0.48* |
| Example 4 group | | 1.68±0.58* |
| Comparative example 1 group | | 1.71±0.52* |
| Comparative example 2 group | | 1.73±0.50* |
| Comparative example 3 group | | 1.70±0.41* |

| | | |
|---|---|---|
| Note: Compared with the blank group, *P<0.01. | | |

**Table 4 Behavioral Scores of Rats after Administration for 14d (x̅ ± s, n=10)**

| Group | | Behavioral score |
|---|---|---|
| | | 14d |
| Blank group | | 9.80±0.42 |
| Model group | | 2.20±0.82* |
| Example 1 | High dose group | 6.68±0.97*^{#} |
| | Medium dose group | 6.65±0.88*^{#} |
| | Low dose group | 5.10±1.06*^{#} |
| Example 2 group | | 5.40±1.03*^{#} |
| Example 3 group | | 5.90±0.97*^{#} |
| Example 4 group | | 6.80±1.05^{*#} |
| Comparative example 1 group | | 3.30±1.42^{*#} |
| Comparative example 2 group | | 4.20±1.40^{*#} |
| Comparative example 3 group | | 3.70±1.34^{*#} |

| | | |
|---|---|---|
| Note: Compared with the blank group, *P<0.01; Compared with the model group, ^{#}P<0.01. | | |

The results of behavioral scores of facial neuritis showed that among the comparison between the blank group and model group, three dose groups (high, medium, low) in Example 1, Example 2-4 groups, and Comparative example 1-4 groups after administration on 0d, the difference was statistically significant (P<0.01), which proved that the model was successful; on the 14th day of administration, among the model group and blank group, three dose groups (high, medium and low) in Example 1, Example 2-4 groups, and Comparative example 1-4 groups respectively, the difference was statistically significant (P<0.01).

### 4.2 Expression of VEGFb, VEGFR1 and VEGFR2 protein in lateral nerves of rats after administration for 14 days

**Table 5 Expression of VEGFb Protein in Lateral Nerves of Rats after Administration for 14 Days (x̅ ± s, n=10)**

| Group | | VEGFb/Actin |
|---|---|---|
| Blank group | | 1.72±0.07 |
| Model group | | 0.76±0.06 |
| Example 1 | High dose group | 1.59±0.15*^{#} |
| | Medium dose group | 1.58±0.14*^{#} |
| | Low dose group | 1.19±0.13*^{#} |
| Example 2 group | | 1.25±0.09*^{#} |
| Example 3 group | | 1.46±0.11*^{#} |
| Example 4 group | | 1.62±0.11^{*#} |
| Comparative example 1 group | | 0.92±0.12^{*%} |
| Comparative example 2 group | | 1.06±0.14^{*#} |
| Comparative example 3 group | | 0.98±0.11^{*#} |

| | | |
|---|---|---|
| Note: Compared with the blank group, *P<0.01; Compared with the model group, ^{#}P<0.01 ,^{%}P<0.05. | | |

**Table 6 Expression of VEGFR1 Protein in Lateral Nerves of Rats after Administration for 14 Days (x̅ ± s, n=10)**

| Group | | VEGFR1/Actin |
|---|---|---|
| Blank group | | 1.58±0.05 |
| Model group | | 0.68±0.04 |
| Example 1 | High dose group | 1.36±0.14*^{#} |
| | Medium dose group | 1.35±0.10*^{#} |
| | Low dose group | 1.05±0.05*^{#} |
| Example 2 group | | 1.13±0.15*^{#} |
| Example 3 group | | 1.27±0.15*^{#} |
| Example 4 group | | 1.38±0.09^{*#} |
| Comparative example 1 group | | 0.86±0.15^{*#} |
| Comparative example 2 group | | 0.95±0.12^{*#} |
| Comparative example 3 group | | 0.89±0.13^{*#} |

| | | |
|---|---|---|
| Note: Compared with the blank group, *P<0.01; Compared with the model group, ^{#}P<0.01. | | |

**Table 7 Expression of VEGFR2 Protein in Lateral Nerves of Rats after Administration for 14 Days (x̅ ± s, n=10)**

| Group | | VEGFR2/Actin |
|---|---|---|
| Blank group | | 1.56±0.03 |
| Model group | | 0.61±0.05 |
| Example 1 | High dose group | 1.53±0.15*^{#} |
| | Medium dose group | 1.54±0.13*^{#} |
| | Low dose group | 1.27±0.12*^{#} |
| Example 2 group | | 1.36±0.14*^{#} |
| Example 3 group | | 1.43±0.13*^{#} |
| Example 4 group | | 1.55±0.08*^{#} |
| Comparative example 1 group | | 0.99±0.08*^{#} |
| Comparative example 2 group | | 1.09±0.05*^{#} |
| Comparative example 3 group | | 1.04±0.08*^{#} |

| | | |
|---|---|---|
| Note: Compared with the blank group, *P<0.01; Compared with the model group, ^{#}P<0.01. | | |

To sum up, according to the pharmacodynamic experimental results, the traditional Chinese medicine composition of the present invention can obviously relieve the facial paralysis related symptoms of facial neuritis, significantly improve the expression of VEGFb, VEGFR1 and VEGFR2 proteins in the lateral nerves of rats after operation, promote the recovery and regeneration of axons after injury, and effectively treat facial neuritis.

### 4.3 Study on nerve conduction velocity of lateral nerve potential of rats after administration for 14 days

After administration for 14 days, rats were anesthetized by intraperitoneal injection, and facial nerves were separated. Stimulating electrodes and recording electrodes were placed along the separated facial nerves from proximal to distal. Nerve conduction velocity CV (m·s⁻¹) = distance between stimulating electrodes and recording electrodes/conduction time. The mean value was calculated by repeated measurement for 10 times.

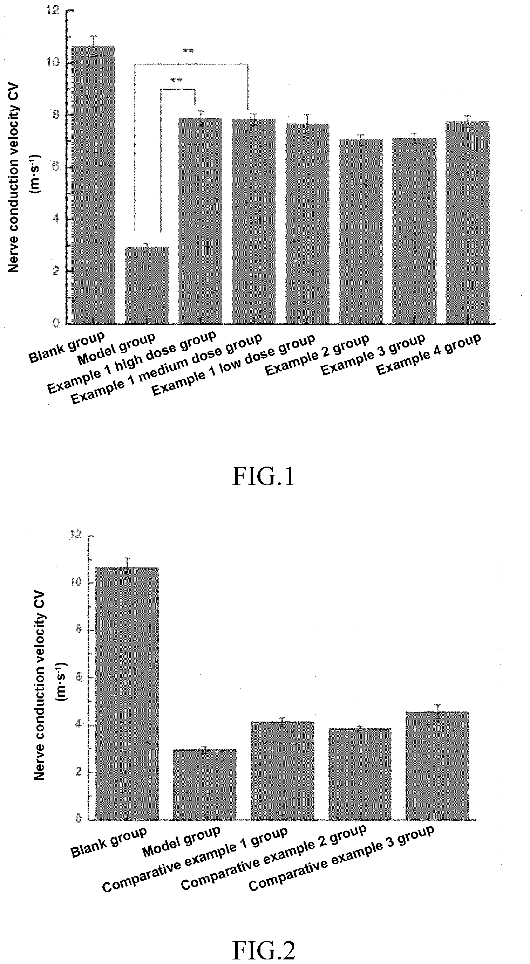

As shown in Fig. 1, after administration for 14 days, the rats in each group were compared in potential nerve conduction velocity test, which showed that among the comparison between the model group and blank group, three dose groups (high, medium and low) in Example 1, and Example 2-4 groups respectively, the difference was statistically significant (P<0.01).

Fig. 2 is a comparison of facial nerve conduction velocity of rats in Comparative examples 1-3, model group and blank group. Comprehensive comparison shows that the traditional Chinese medicine composition of the examples of the present invention can stimulate electrical conduction of facial nerve of rats and effectively treat facial neuritis.

### 4.4 Expression of Beclin1 protein and P62 protein in facial nerve tissue of rats

After administration for 14 days, the expression of Beclin1 protein in facial nerve tissue was detected by immunohistochemistry and P62 protein in facial nerve tissue was detected by RT-PCR.

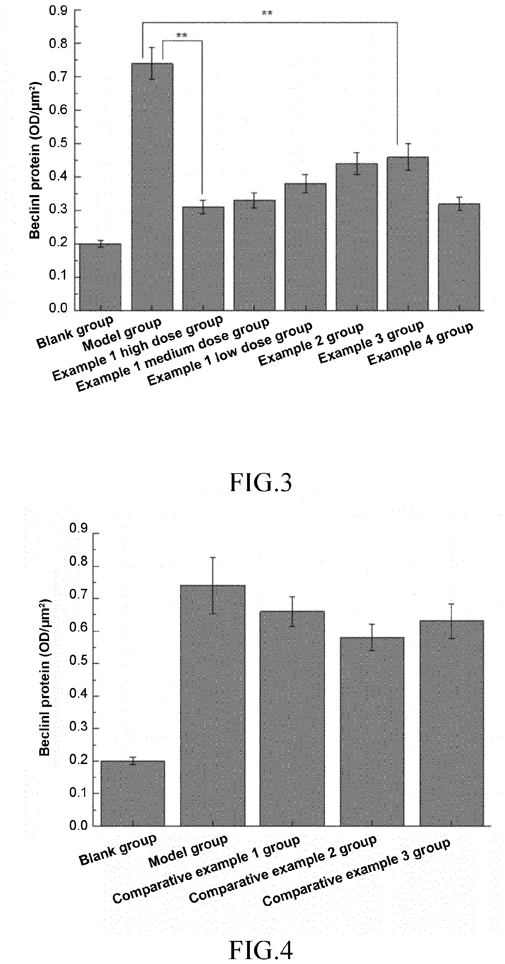

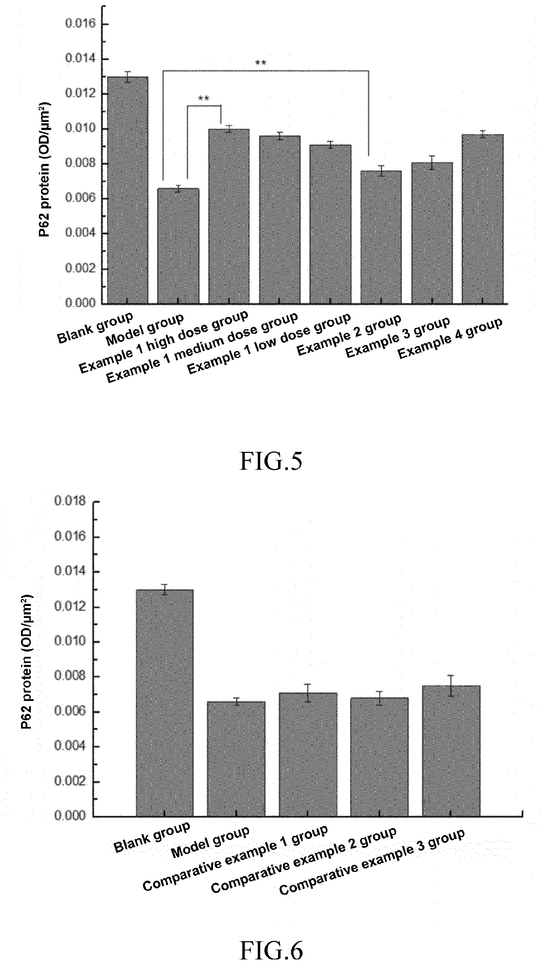

Fig. 3, Fig. 4, Fig. 5 and Fig. 6 show the expression levels of Beclin1 protein and P62 protein in facial nerve tissue of rats after administration for 14 days. The results showed that the traditional Chinese medicine composition of the present invention can reduce the expression of Beclin1 protein in the facial nerve tissue of rats, increase the level of P62 protein, participate in the regulation mechanism of autophagy, and promote the recovery of facial nerve function. Among the comparison between the model group and blank group, three dose groups (high, medium and low) in Example 1, and Example 2-4 groups respectively, the difference was statistically significant (P<0.01).

To sum up, through the behavior score of rats, it can be seen that the traditional Chinese medicine composition of the present invention can obviously relieve facial paralysis, dyskinesia and other related symptoms of facial neuritis of rats; can significantly increase the expression of VEGFb, VEGFR1 and VEGFR2 proteins in the lateral nerve of rats after operation, promote the recovery and regeneration of axons after injury, stimulate the electrical conduction of facial nerve in rats, improve the electrical conduction velocity of facial nerve, reduce the expression of Beclin1 protein in facial nerve tissue of rats, increase the level of P62 protein, and promote the recovery of facial nerve function. Therefore, the traditional Chinese medicine composition of the present invention has the effect of treating facial neuritis and can improve the related symptoms of facial neuritis.

## Claims

1. Use of a traditional Chinese medicine composition in the preparation of a medicament for treating neuritis, wherein the traditional Chinese medicinal composition comprises Notopterygii Rhizoma et Radix, Angelicae Pubescentis Radix, Poria, Saposhnikoviae Radix, Schizonepetae Herba, Chuanxiong Rhizoma, Platycodonis Radix, Bupleuri Radix, Peucedani Radix, Aurantii Fructus and Glycyrrhizae Radix et Rhizoma.

2. The use according to Claim 1, **characterized in that** the traditional Chinese medicine composition comprises the following raw materials:
Schizonepetae Herba 50-100 parts by weight, Saposhnikoviae Radix 50-100 parts by weight, Notopterygii Rhizoma et Radix 50-100 parts by weight
Angelicae Pubescentis Radix 50-100 parts by weight, Bupleuri Radix 50-100 parts by weight, Peucedani Radix 50-100 parts by weight
Chuanxiong Rhizoma 50-100 parts by weight, Aurantii Fructus 50-100 parts by weight, Poria 50-100 parts by weight
Platycodonis Radix 50-100 parts by weight, and Glycyrrhizae Radix et Rhizoma 5-50 parts by weight.

3. The use according to Claim 2, **characterized in that** the traditional Chinese medicine composition comprises the following raw materials:
Schizonepetae Herba 75 parts by weight, Saposhnikoviae Radix 75 parts by weight, Notopterygii Rhizoma et Radix 75 parts by weight
Angelicae Pubescentis Radix 75 parts by weight, Bupleuri Radix 75 parts by weight, Peucedani Radix 75 parts by weight
Chuanxiong Rhizoma 75 parts by weight, Aurantii Fructus 75 parts by weight, Poria 75 parts by weight
Platycodonis Radix 75 parts by weight, and Glycyrrhizae Radix et Rhizoma 25 parts by weight.

4. The use according to Claim 1, **characterized in that** the neuritis comprises facial neuritis, trigeminal neuritis and polyneuritis.

5. The use according to Claim 1, **characterized in that** the neuritis is caused by one or more of infection, poisoning, genetic defect, nutritional disorder, immune damage, metabolic disorder, endocrine disorder, congenital malformation, blood circulation disorder, abnormal hyperplasia, wind-cold and trauma.

6. The use according to Claim 1, **characterized in that** the traditional Chinese medicine composition can improve the symptoms of neuritis, and the symptoms include irritation symptoms and/or destruction symptoms, wherein the irritation symptoms show pain and numbness, and the destruction symptoms show weakness and paralysis.

7. The use according to Claim 1, **characterized in that** the traditional Chinese medicine composition can improve the symptoms of facial neuritis, including shallow nasolabial groove, distortion of commissure, air leakage and salivation in speech, loss of facial random movements, large palpebral fissure, inability to successfully complete frowning, closing eyes, whistling and other movements, and loss of one or more facial expressions.

8. The use according to any one of Claims 1-7, **characterized in that** the traditional Chinese medicine composition can be prepared into a preparation with pharmaceutically acceptable excipients; and the preparation comprises granules, mixtures, tablets, capsules, pills, dustpowders, powders, syrups, microcapsules and ointments.

9. Use of JINGFANG Preparation in drugs for treating neuritis.

10. Use of JINGFANG Preparation in drugs for treating facial neuritis.
